# EUROPÄISCHE PATENTANMELDUNG

(11) **EP 1 872 822 A1**
(43) Veröffentlichungstag der Anmeldung: **02.01.2008**
(21) Anmeldenummer: 06013344.4
(22) Anmeldetag: 28.06.2006
(51) Int. Cl.: A61M 37/00, A01K 11/00

(54) **Anordnung zum wiederholten Aufstechen einer Haut mit einem Handgerät, Nadelmodul und Verfahren zum Montieren des Handgerätes**

(71) Anmelder: MediUm-TECH Medizingeräte GmbH, 14167 Berlin (DE)
(72) Erfinder: Kluge, Jörn, 14513 Teltow (DE); Plückhahn, Kristian, 12437 Berlin (DE); Podolski, Denis, 10961 Berlin (DE); Halim, Ryanto Chandra, 10551 Berlin (DE)
(74) Vertreter: Bittner, Thomas L.

(57) **Zusammenfassung**

Die Erfindung bezieht sich auf eine Anordnung zum wiederholten Aufstechen einer Haut, mit einem Handgerät (1), welches ein Antriebsmodul (2) mit einer Antriebsvorrichtung (4), die konfiguriert ist, um eine Antriebskraft zu erzeugen, und ein Nadelmodul. (3) aufweist, in dem eine Stechvorrichtung (6) in Längsrichtung des Nadelmoduls (3) verlagerbar angeordnet ist und welches an das Antriebsmodul (2) lösbar gekoppelt ist, sodaß die Antriebskraft in die Stechvonichtung (6) eingeleitet ist, wobei das Nadelmodul (3) Kennungsmittel (14) aufweist, die mit einer den Kennungsmitteln (14) zugeordneten und wahlweise von dem Antriebsmodul (2) umfaßten Auswertevorrichtung (13) auswertbar sind. Weiterhin betrifft die Erfindung ein Nadelmodul sowie ein Verfahren zum Montieren eines Handgerätes.

## Beschreibung

Die Erfindung bezieht sich auf eine Anordnung zum wiederholten Aufstechen einer Haut mit einem Handgerät, welches ein Antriebsmodul mit einer Antriebsvorrichtung und ein Nadelmodul aufweist, sowie ein Nadelmodul für eine solche Anordnung und ein Verfahren zum Montieren des Handgerätes.

### Hintergrund der Erfindung

Anordnungen zum wiederholten Aufstechen einer Haut mit einem Handgerät, welches ein Antriebsmodul mit einer Antriebsvorrichtung, die konfiguriert ist, um eine Antriebskraft zu erzeugen, und ein Nadelmodul aufweist, in dem eine Stechvorrichtung in Längsrichtung des Nadelmoduls verlagerbar angeordnet ist und welches an das Antriebsmodul lösbar gekoppelt ist, sodaß die Antriebskraft in die Stechvorrichtung eingeleitet ist, werden in verschiedenen Anwendungen genutzt, um eine Haut für das Einbringen eines Wirkstoffes in die Haut aufzustechen. Mit Hilfe der Antriebskraft wird die Stechvorrichtung repetierend vor und zurück bewegt. Bei dem einzubringenden. Wirkstoff kann es sich beispielsweise um einen Farbstoff, einen kosmetischen Wirkstoff oder einen medizinischen Wirkstoff handeln. In Verbindung mit dem Farbstoff wird das Handgerät zum Beispiel als Tattoo- und Permanent-Make-up-Handgerät verwendet. Bei der medizinischen Anwendung können mit dem Handgerät Impfstoffe oder andere medizinisch wirksame Stoffe in die Haut eingebracht werden.

Aus dem Dokument DE 299 19 199 ist bekannt, bei derartigen Anordnungen zum wiederholten Aufstechen der Haut einen modulartigen Aufbau derart vorzusehen, daß das Handgerät mit einem Antriebsmodul und einem hieran lösbar zu befestigenden Nadelmodul zu bilden. Um eine korrekte Anwendung des Handgerätes sicherzustellen, ist es von besonderer Bedeutung, daß das Antriebsmodul, welches über die Antriebsvorrichtung verfügt, beispielsweise einen Elektromotor, mit dem richtigen Nadelmodul gekoppelt wird.

### Zusammenfassung der Erfindung

Aufgabe der Erfindung ist es deshalb, eine verbesserte Anordnung zum wiederholten Aufstechen einer Haut mit einem Handgerät, ein verbessertes Nadelmodul für die Anordnung sowie ein verbessertes Verfahren zum Montieren des Handgerätes zu schaffen, die eine ordnungsgemäße Nutzung von Antriebsmodul und Nadelmodul sicherstellen.

Diese Aufgabe wird erfindungsgemäß durch eine Anordnung zum wiederholten Aufstechen einer Haut nach dem unabhängigen Anspruch 1, ein Nadelmodul für die Anordnung nach dem unabhängigen Anspruch 14 sowie ein Verfahren zum Montieren des Handgerätes nach dem unabhängigen Anspruch 15 gelöst. Vorteilhafte Ausgestaltungen der Erfindung sind Gegenstand abhängiger Ansprüche.

Die Erfindung umfaßt den Gedanken, eine Anordnung zum wiederholten Aufstechen einer Haut vorzusehen, bei der ein Handgerät, welches ein Antriebsmodul mit einer Antriebsvorrichtung, die konfiguriert ist, um eine Antriebskraft zu erzeugen, und ein Nadelmodul aufweist, in dem eine Stechvorrichtung in Längsrichtung des Nadelmoduls verlagerbar angeordnet ist und welches an das Antriebsmodul lösbar gekoppelt ist, so daß die Antriebskraft in die Stechvorrichtung eingeleitet ist, an dem Nadelmodul Kennungsmittel vorzusehen, die mit einer den Kennungsmitteln zugeordneten und wahlweise von dem Antriebsmodul umfaßten Auswertevorrichtung auswertbar sind. Auf diese Weise können dem Nadelmodul bei der Herstellung beliebige Kennungsinformationen beigegeben werden, die dann im Rahmen der Nutzung des Nadelmoduls in Verbindung mit dem Antriebsmodul auswertbar sind. Je nach Anwendungsfall kann das Nadelmodul mit Hilfe der Kennungsmittel individuell konfiguriert werden. Es ist so erleichtert, die Einhaltung von Sicherheitsvorschriften zu gewährleisten. Darüber hinaus ist es mit der Erfindung einfacher möglich, eine zweckentfremdende Nutzung des Nadelmoduls zu verhindern.

Eine bevorzugte Weiterbildung der Erfindung sieht vor, daß die Kennungsmittel mit Hilfe mindestens eines der folgenden Auswerteverfahren von der Auswertevorrichtung auswertbar sind: kabellose Datenübertragung wie Funkdatenübertragung, Infrarot-Datenübertragung und Bluetooth-Datenübertragung, induktive Auswertung, kapazitive Auswertung, optische Auswertung und mechanische Auswertung. Vorteil einer mechanischen Auswertung ist die einfache Ausgestaltung. So kann an dem Antriebsmodul beispielsweise eine Vertiefung vorgesehen sein, die hinsichtlich ihrer Formgestaltung im wesentlichen an einen Vorsprung an dem Nadelmodul angepaßt ist, so daß nur ein Nadelmodul mit derart ausgestattetem Vorsprung mit dem zugehörigen Antriebsmodul koppelbar ist. Der Vorsprung an dem Nadelmodul bildet das Kennungsmittel, welches mittels der zugeordneten Vertiefung (Auswertevorrichtung) an dem Antriebsmodul ausgewertet wird. Die Gestaltungsmöglichkeiten hinsichtlich der Auswertung der Kennungsmittel sind allerdings bei der Nutzung einer Datenübertragung besonders vielfältig.

Bei einer zweckmäßigen Ausgestaltung der Erfindung kann vorgesehen sein, daß die Kennungsmittel elektronische Kennungsmittel mit hierin gespeicherten Daten aufweisen, die von der Auswertevorrichtung auswertbar sind. Die Nutzung eines elektronischen Kennungsmittels hat den Vorteil, daß die hierin gespeicherten Daten beim Auswerten in vielfältiger Art und Weise verarbeitet werden können. Beispielsweise können hieraus Steuerungsdaten für den Betrieb des Handgerätes abgeleitet werden.

Eine vorteilhafte Ausführungsform der Erfindung sieht vor, daß die Auswertevorrichtung ein den elektronischen Kennungsmitteln zugeordnetes Kommunikationsmodul umfaßt, mit dem die gespeicherten Daten aus den elektronischen Kennungsmitteln ausgelesen und wahlweise weitere Daten in den elektronischen Kennungsmitteln gespeichert werden können. Bei dem Kommunikationsmodul handelt es sich beispielsweise um eine für die elektronischen Kennungsmittel geeignete Sende-Empfangseinheit, sodaß elektronische Daten ausgestaucht werden können.

Eine bevorzugte Fortbildung der Erfindung sieht vor, daß die elektronischen Kennungsmittel einen. RFID-Chip (RFID - Radio Frequenz Identifikation) umfassen. Die RFID-Technologie ist als solche bekannt und ermöglicht es, beliebige Informationen auf dem RFID-Chip zu speichern, die mit einem zugeordneten Auswertemodul ausgelesen werden kann.

Eine bevorzugte Weiterbildung der Erfindung sieht vor, daß die Auswertevorrichtung konfiguriert ist, um die Kennungsmittel automatisch auszuwerten, wenn das Nadelmodul an das Antriebsmodul gekoppelt ist.

Bei einer zweckmäßigen Ausgestaltung der Erfindung kann vorgesehen sein, daß die Kennungsmittel zumindest eine der folgenden, von der Auswertevorrichtung auswertbaren und dem Nadelmodul zuordenbaren Informationen liefernd gebildet sind: Seriennummer, für das Nadelmodul nutzbarc(s) Antriebsmodul(e), Nutzungsdauer des Nadelmoduls, Art der Stechvorrichtung und erlaubte Betriebsparameter wie Stechfrequenz und Betriebsarten.

Eine vorteilhafte Ausführungsform der Erfindung sieht vor, daß eine Steuervorrichtung gebildet ist, die konfiguriert ist, um einen Betrieb des Handgerätes zu steuern.

Eine bevorzugte Fortbildung der Erfindung sieht vor, daß die Steuervorrichtung in zumindest einem der folgenden Geräteteile implementiert ist: Handgerät und ein an das Handgerät gekoppeltes Steuergerät. Das Steuergerät wird in Verbindung mit Handgeräten zum Hautaufstechen üblicherweise dazu genutzt, Betriebsparameter zu regeln und eine Spannungsversorgung zur Verfügung zu stellen. Das Steuergerät selbst verfügt häufig über eine Anzeigeeinrichtung, Eingabemittel wie Drehknöpfe oder Tastenfelder.

Eine bevorzugte Weiterbildung der Erfindung sieht vor, daß die Auswertevorrichtung oder wahlweise die Steuervorrichtung konfiguriert sind, um den Betrieb des Handgerätes zu unterbinden, wenn beim Auswerten der Kennungsmittel festgestellt wird, daß das Nadelmodul für eine Nutzung mit dem Antriebsmodul nicht zugelassen ist. Wenn bei der Auswertung der Kennungsmittel festgestellt wird, daß das Nadelmodul für eine Nutzung mit dem Antriebsmodul nicht zugelassen ist, beispielsweise anhand der ausgewerteten Seriennummer, verhindern die Auswertevorrichtung oder wahlweise die Steuervorrichtung eine Nutzung des Handgerätes, was beispielsweise dadurch erreicht wird, daß eine Spannungsversorgung der Antriebsvorrichtung unterbrochen wird.

Bei einer zweckmäßigen Ausgestaltung der Erfindung kann vorgesehen sein, daß die Auswertevorrichtung oder wahlweise die Steuervorrichtung konfiguriert sind, um den Betrieb des Handgerätes der mindestens einen von der Auswertevorrichtung auswertbaren und dem Nadelmodul zuordenbaren Information entsprechend zu steuern.

Eine vorteilhafte Ausführungsform der Erfindung sieht vor, daß das Handgerät als eines der folgenden Handgeräte ausgeführt ist: Permanent-Make-up- und Tattoo-Handgerät, Wirkstoffabgabe-Handgerät und Hautimpf-Handgerät.

Eine bevorzugte Fortbildung der Erfindung sieht vor, daß das Nadelmodul ein sterilisiertes Einweg-Nadelmodul ist. Unabhängig von der Ausführung als Einwegmodul kann das Nadelmodul so gestaltet sein, dass es als Gesamtmodul oder in Form einzelner Elemente, beispielsweise als Gehäuse und Nadel oder Nadelsystem, an dem Antriebsmodul montierbar und von diesem Lösbar ist.

Nachfolgend werden vorteilhafte Ausgestaltungen des erfindungsgemäßen Verfahrens näher beschrieben.

Eine bevorzugte Weiterbildung der Erfindung sieht vor, daß die Kennungsmittel mit Hilfe mindestens eines der folgenden Auswerteverfahren von der Auswertevorrichtung ausgewertet werden: kabellose Datenübertragung wie Funkdatenübertragung, Infrarot-Datenübertragung und Bluetooth-Datenübertragung, induktive Auswertung, kapazitive Auswertung, optische Auswertung und mechanische Auswertung.

Bei einer zweckmäßigen Ausgestaltung der Erfindung kann vorgesehen sein, daß die Kennungsmittel elektronische Kennungsmittel mit hierin gespeicherten Daten aufweisen, die von der Auswertevorrichtung ausgewertet werden.

Eine vorteilhafte Ausführungsform der Erfindung sieht vor, daß die Auswertevorrichtung ein den elektronischen Kennungsmitteln zugeordnetes Kommunikationsmodul umfaßt, mit dem beim Auswerten der elektronischen Kennungsmittel die gespeicherten Daten zumindest teilweise aus den elektronischen Kennungsmitteln ausgelesen und wahlweise weitere Daten in den elektronischen Kennungsmitteln gespeichert werden. So kann in einer Ausführung vorgesehen sein, daß in die elektronischen Kennungsmittel eine Information über eine erfolgte Benutzung des Nadelmoduls geschrieben wird, beispielsweise mittels Setzen eines digitalen Parameters. Auf diese Weise kann zum Beispiel ein Versuch der nochmaligen Benutzung des dann vielleicht schon verschmutzten Nadelmoduls unterbunden werden, wenn die elektronischen Kennungsmittel beim nochmaligen Ankoppeln des Nadelmoduls ausgelesen werden. daß beim Auswerten der elektronischen Kennungsmittel Daten aus einem von den elektronischen Kennungsmitteln umfaßten RFID-Chip ausgewertet werden.

Eine bevorzugte Weiterbildung der Erfindung sieht vor, daß die Auswertevorrichtung die Kennungsmittel automatisch auswertet, wenn das Nadelmodul an das Antriebsmodul gekoppelt wird.

Bei einer zweckmäßigen Ausgestaltung der Erfindung kann vorgesehen sein, daß die Kennungsmittel zumindest eine der folgenden, von der Auswertevorrichtung auswertbaren und dem Nadelmodul zuordenbaren Informationen liefern: Seriennummer, für das Nadelmodul nutzbare(s) Antriebsmodul(e), Nutzungsdauer des Nadelmoduls, Art der Stechvorrichtung und erlaubte Betriebsparameter wie Stechfrequenz und Betriebsarten. Als Stechvorrichtung sind insbesondere Einzelnadeln oder Nadelsysteme mit mehreren Nadeln nutzbar.

Eine vorteilhafte Ausführungsform der Erfindung sieht vor, daß durch die Auswertevonichtung oder wahlweise die Steuervorrichtung der Betrieb des Handgerätes unterbunden wird, wenn beim Auswerten der Kennungsmittel festgestellt wird, daß das Nadelmodul für eine Nutzung mit dem Antriebsmodul nicht zugelassen ist.

Eine bevorzugte Fortbildung der Erfindung sieht vor, daß die Auswertevorrichtung oder wahlweise die Steuervorrichtung den Betrieb des Handgerätes der mindestens einen von der Auswertevorrichtung auswertbaren und dem Nadelmodul zuordenbaren Information entsprechend steuert. Eine Steuerung der auswertbaren Informationen entsprechend bedeutet beispielsweise eine Beschränkung des Betriebs des Handgerätes auf vorgegebene Betriebsarten, die zum Beispiel durch eine bestimmte Repetierfrequenz (Stechfrequenz) oder einen Frequenzbereich für die Stechvorrichtung oder eine Einstechtiefe charakterisiert sind, nämlich Parameter, die für den Betrieb des Handgerätes durch den Benutzer einstellbar sind, bevorzugt mittels des Steuergerätes.

### Beschreibung bevorzugter Ausführungsbeispiele der Erfindung

Die Erfindung wird im folgenden anhand von bevorzugten Ausführungsbeispiclen unter Bezugnahme auf eine Figur näher erläutert.

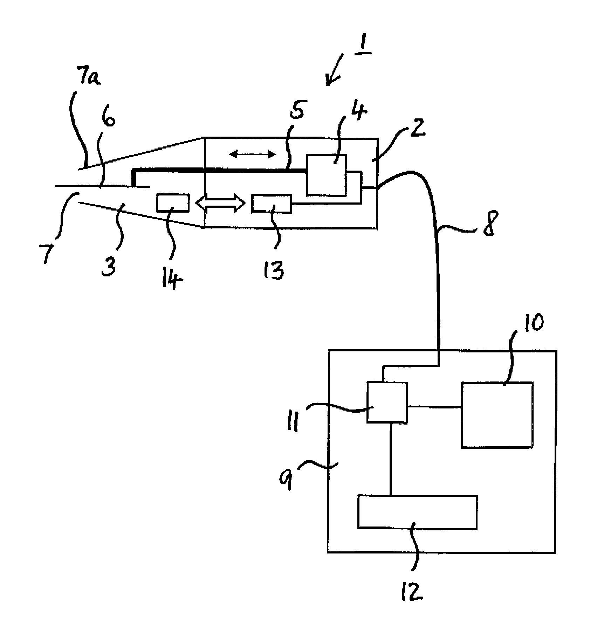

Die einzige Figur zeigt eine schematische Anordnung mit einem Handgerät 1 zum wiederholten Aufstechen einer Haut. Das Handgerät 1 umfaßt ein Antriebsmodul 2 sowie ein hieran lösbar gekoppeltes Nadelmodul 3. In dem Antriebsmodul 2 ist ein Elektromotor 4 als Antriebsvorrichtung vorgesehen, der über einen Kopplungsmechanismus 5, welcher seinerseits bevorzugt einen Taumelscheibenmechanismus umfaßt, an eine Nadel 6 koppelt. Die Nadel 6 bildet eine Stechvorrichtung zum Hautaufstechen, welche in anderen Ausführungen auch von einem System mehrerer Nadeln gebildet ist. Mit Hilfe des Elektromotors 4 und des Kopplungsmechanismus 5 wird eine repetierende Antriebsbewegung erzeugt, die über den Kopplungsmechanismus 5 auf die Nadel 6 eingeleitet wird, so daß die Nadel 6 durch eine Öffnung 7 des Nadelmoduls 3 hin und her bewegt wird. Üblicherweise ist die Nadel 6 mittels eines Nadelschaftes (nicht dargestellt) an den Kopplungsmechanismus 5 gekoppelt. Mit an die Öffnung 7 grenzenden Gehäuseabschnitten 7a des Nadelmoduls 3 ist eine Nadeldüse gebildet.

Das Nadelmodul 3 ist lösbar mit dem Antriebsmodul 2 verbunden, beispielsweise mittels einer Schraub-, einer Steck- oder einer Klemmverbindung. Auf diese Weise ist es ermöglicht, daß vorzugsweise als Einwegmodul ausgeführte Nadelmodul 3 nach einer Nutzung von dem Antriebsmodul 2 zu lösen und auszutauschen. Hierbei ist das Nadelmodul 3 so ausgeführt, daß es als Gesamtmodul von dem Antriebsmodul 2 gelöst werden kann. Das Nadelmodul 3 wird vorzugsweise als sterilisiertes Bauteil in einer geeigneten Verpackung zur Verfügung gestellt.

Über eine Verbindungsleitung 8 ist das Handgerät 1 mit einem Steuergerät 9 verbunden. Mit Hilfe der Verbindungsleitung 8 erfolgt insbesondere der Anschluß des Elektromotors 4 an eine Spannungsversorgung (nicht dargestellt). Darüber hinaus umfaßt die Verbindungsleitung 8 in der dargestellten Ausführungsform ein oder mehrere Leitungen zur Übertragung elektronischer Daten. In alternativen Ausgestaltungen kann das Handgerät 1 auch über eine kabellose Datenverbindung mit dem Steuergerät 9 verbunden sein. Eine Spannungsversorgung des Handgerätes 1, kann in einer Ausgestaltung mit Hilfe eines in das Handgerät 1 integrierten Akkus ausgeführt sein.

In dem Steuergerät 9 sind eine Anzeigenvorrichtung 10, eine Steuervorrichtung 11 sowie eine Bedienvorrichtung 12 gebildet. Weitere Bauteile oder Baugruppen können in dem Steuergerät 9 vorgesehen sein, werden hier aber zur Vereinfachung der Darstellung weggelassen. Mit Hilfe der Bedienvorrichtung 12 kann ein Benutzer Vorgaben für den Betrieb des Handgerätes 1 eingeben, beispielsweise über ein Tastenfeld oder mit Hilfe von Drehknöpfen. Über die Anzeigevorrichtung 11 werden Informationen zum Betrieb des Handgerätes 1 angezeigt, beispielsweise eine genutzte Betriebsart, eine gewählte Stechfrequenz oder dergleichen. Auch die Anzeige von Informationen betreffend das aktuell verwendete Nadelmodul 3 kann vorgesehen sein. Die Steuervorrichtung 10 dient insbesondere zum Steuern des Betriebs des Handgerätes 1 aber auch zur Koordination der Anzeige auf der Anzeigevorrichtung 11.

Gemäß der schematischen Darstellung in der Figur ist in dem Antriebsmodul 2 eine Auswertevorrichtung 13 gebildet, die genutzt wird, um Kennungsmittel 14 an dem Nadelmodul 3 auszuwerten. Die Kennungsmittel 14 und die Auswertevorrichtung 13 können mechanisch, optisch, kapazitiv oder induktiv miteinander gekoppelt sein. Beim Ankoppeln des Nadelmoduls 3 werden die Kennungsmittel 14 automatisch oder vom Benutzer initiiert, beispielsweise mittels Bedienung eines Tasters, von der Auswertevorrichtung 13 ausgewertet. Von der Auswertevorrichtung 13 werden dann bei der dargestellten Ausführungsform Datensignale an die Steuervorrichtung 10 übermittelt, die Informationen anzeigen, welche die Auswertevorrichtung 13 aus den Kennungsmitteln 14 abgeleitet hat. Beispielsweise kann es sich hierbei um ein Datensignal handeln, welches anzeigt, daß das Nadelmodul 3 passend zu dem Antriebsmodul 2 ist. Ein solches Signal kann zum Beispiel mit Hilfe eines an dem Antriebsmodul 2 angebrachten Tastschalters erzeugt werden, welcher bedient wird, wenn das Nadelmodul 3 an das Antriebsmodul 2 gekoppelt wird, indem beispielsweise ein Vorsprung (nicht dargestellt) an dem Nadelmodul 3 beim Koppeln auf den Tastschalter drückt, welcher in die Auswertevorrichtung 13 integriert ist.

Bevorzugte Ausgestaltungen sehen alternativ oder ergänzend vor, daß die Kennungsmittel 14 ein elektronisches Speichermedium umfassen, beispielsweise einen RFID-Chip, in dem elektronische Daten gespeichert sind, die ihrerseits zumindest zum Teil mit Hilfe der Auswertevorrichtung 13 ausgewertet werden können.

In Abhängigkeit von den von der Auswertevorrichtung 13 erhaltenen Datensignalen steuert die Steuervorrichtung 10 dann den Betrieb des Handgerätes 1. Beispielsweise kann der Betrieb des Handgerätes 1 freigegeben oder gesperrt werden, je nach dem ob die Datensignale das Koppeln eines korrekten Nadelmoduls oder eines nicht mit dem Antriebsmodul 2 nicht zugelassenen Nadelmoduls anzeigen. Des weiteren können die mittels der Kennungsmittel 14 zur Verfügung gestellten Informationen zugelassene Betriebsarten des Nadelmoduls 3 oder Nutzungsbeschränkungen des Nadelmoduls 3 umfassen, beispielsweise hinsichtlich einer Nutzungsdauer. Die Steuereinrichtung 10 ist konfiguriert, um die von der Auswertevorrichtung 13 übersandten Datensignale dann entsprechend bei der Steuerung des Betriebs des Handgerätes 1 zu berücksichtigen.

Alternativ zu der Ausführungsform in der Figur kann die Steuervorrichtung 10 zumindest teilweise in das Handgerät 1 integriert sein, beispielsweise in die Auswertevorrichtung 13. Zu diesem Zweck ist zum Beispiel in Mikroprozessor (nicht dargestellt) in das Handgerät 1 integriert, nämlich in das Antriebsmodul 2. Die Nutzung der aus den Kennungsmitteln 14 abgeleiteten Informationen für die Steuerung des Handgerätes 1 kann dann zumindest teilweise in dem Handgerät 1 selbst erfolgen.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Anordnung zum wiederholten Aufstechen einer Haut, mit einem Handgerät (1), welches ein Antriebsmodul (2) mit einer Antriebsvorrichtung (4), die konfiguriert ist, um eine Antriebskraft zu erzeugen, und ein Nadelmodul (3) aufweist, in dem eine Stechvorricbtung (6) in Längsrichtung des Nadelmoduls (3) verlagerbar angeordnet ist und welches an das Antriebsmodul (2) lösbar gekoppelt ist, sodaß die Antriebskraft in die Stechvorrichtung (6) eingeleitet ist, wobei das Nadelmodul (3) Kennungsmittel (14) aufweist, die mit einer den Kennungsmitteln (14) zugeordneten und wahlweise von dem Antriebsmodul (2) umfaßten Auswertevorrichtung (13) auswertbar sind.

2. Anordnung nach Anspruch 1, **dadurch gekennzeichnet, daß** die Kennungsmittel (14) mit Hilfe mindestens eines der folgenden. Auswerteverfahren von der Auswertevorrichtung (13) auswertbar sind: kabellose Datenübertragung wie Funkdatenübertragung, Infrarot-Datenübertragung und Bluetooth-Datenübertragung, induktive Auswertung, kapazitive Auswertung, optische Auswertung und mechanische Auswertung.

3. Anordnung nach Anspruch 1 oder 2, **dadurch gekennzeichnet, daß** die Kennungsmittel (14) elektronische Kennungsmittel mit hierin gespeicherten Daten aufweisen, die von der Auswertevorrichtung (13) auswertbar sind.

4. Anordnung nach Anspruch 3, **dadurch gekennzeichnet, daß** die Auswertevorrichtung (13) ein den elektronischen Kennungsmitteln (14) zugeordnetes Kommunikationsmodul umfaßt, mit dem die gespeicherten Daten aus den elektronischen Kennungsmitteln ausgelesen und wahlweise weitere Daten in den elektronischen Kennungsmitteln gespeichert werden können.

5. Anordnung nach Anspruch 3 oder 4, **dadurch gekennzeichnet, daß** die elektronischen Kennungsmittel einen RFID-Chip umfassen.

6. Anordnung nach mindestens einem der vorangehenden Ansprüche, **dadurch gekennzeichnet, daß** die Auswertevorrichtung (13) konfiguriert ist, um dic Kennungsmittel (14) automatisch auszuwerten, wenn das Nadelmodul (3) an das Antriebsmodul (2) gekoppelt ist.

7. Anordnung nach mindestens einem der vorangehenden Ansprüche, **dadurch gekennzeichnet, daß** die Kennungsmittel (14) zumindest eine der folgenden, von der Auswertevorrichtung (13) auswertbaren und dem Nadelmodul (3) zuordenbaren Informationen liefernd gebildet sind: Seriennummer, für das Nadelmodul (3) nutzbare(s) Antriebsmodul(e), Nutzungsdauer des Nadelmoduls (3), Art der Stechvorrichtung (6) und erlaubte Betriebsparameter wie Stechfrequenz und Betriebsarten.

8. Anordnung nach mindestens einem der vorangehenden Ansprüche, **dadurch gekennzeichnet, daß** eine Steuervorrichtung (10) gebildet ist, die konfiguriert ist, um einen Betrieb des Handgerätes (1) zu steuern.

9. Anordnung nach Anspruch 8, **dadurch gekennzeichnet, daß** die Steuervorrichtung (10) in zumindest einem der folgenden Geräteteile implementiert ist: Handgerät (1) und ein an das Handgerät (1) gekoppeltes Steuergerät (9).

10. Anordnung nach mindestens einem der vorangehenden Ansprüche, **dadurch gekennzeichnet, daß** die Auswertevorrichtung (13) oder wahlweise die Steuervorrichtung (10) konfiguriert sind, um den Betrieb des Handgerätes (1) zu unterbinden, wenn beim Auswerten der Kennungsmittel (14) festgestellt wird, daß das Nadelmodul (3) für eine Nutzung mit dem Antriebsmodul (2) nicht zugelassen ist.

11. Anordnung nach mindestens einem Anspruch 7 oder mindestens einem der Ansprüche 8 bis 10, soweit auf Anspruch 7 rückbezogen, **dadurch gekennzeichnet, daß** die Auswertevorrichtung (13) oder wahlweise die Steuervorrichtung (10) konfiguriert sind, um den Betrieb des Handgerätes (1) der mindestens einen von der Auswertevorrichtung (13) auswertbaren und dem Nadelmodul (3) zuordenbaren Information entsprechend zu steuern.

12. Anordnung nach mindestens einem der vorangehenden Ansprüche, **dadurch gekennzeichnet, daß** das Handgerät (1) als eines der folgenden Handgeräte ausgeführt ist: Permanent-Make-up- und Tattoo-Handgerät, Wirkstoffabgabe-Handgerät und Hautimpf-Handgerät.

13. Anordnung nach mindestens einem der vorangehenden Ansprüche, **dadurch gekennzeichnet, daß** das Nadelmodul (3) ein sterilisiertes Binweg-Nadelmodul ist.

14. Nadelmodul (3) für eine Anordnung nach einem der vorangehenden Ansprüche, mit einem Nadelmodul-Gehäuse, welches Kopplungsmittel zum lösbaren Koppeln des Nadelmodul-Gehäuses an ein Antriebsmodul (2) mit einer Antriebsvorrichtung (4) aufweist, und einer Stechvorrichtung (6), welche in Längsrichtung verlagerbar in dem Nadelmodul-Gehäuse angeordnet ist, wobei Kennungsmittel (14) gebildet sind, die mit einer den Kennungsmitteln (14) zugeordneten Auswertevorrichtung (13) auswertbar sind.

15. Verfahren zum Montieren eines Handgerätes (1), mit eine Haut wiederholt aufgestochen werden kann, bei dem ein Antriebsmodul (2) mit einer Antriebsvorrichtung (4), die konfiguriert ist, um eine Antriebskraft zu erzeugen, mit einem Nadelmodul (3), in dem eine Stechvorrichtung (6) in Längsrichtung des Nadelmoduls (3) verlagerbar angeordnet ist, lösbar gekoppelt wird, sodaß die Antriebskraft in die Stechvorrichtung (6) einleitbar ist, und bei dem Kennungsmittel (14) von einer den Kennungsmitteln (14) zugeordneten und wahlweise in dem Antriebsmodul (2) angeordneten Auswertevorrichtung (13) ausgewertet werden.

16. Verfahren nach Anspruch 15, **dadurch gekennzeichnet, daß** die Kennungsmittel (14) mit Hilfe mindestens eines der folgenden Auswerteverfahren von der Auswertevorrichtung (13) ausgewertet werden: kabellose Datenübertragung wie Funkdatenübertragung, Infrarot-Daternübertragung und Bluetooth-Datenübertragung, induktive Auswertung, kapazitive Auswertung, optische Auswertung und mechanische Auswertung.

17. Verfahren nach Anspruch 15 oder 16, **dadurch gekennzeichnet, daß** die Kennungsmittel (14) elektronische Kennungsmittel mit hierin gespeicherten Daten aufweisen, die von der Auswertevorrichtung (13) ausgewertet werden.

18. Verfahren nach Anspruch 17, **dadurch gekennzeichnet, daß** die Auswertevorrichtung (13) ein den elektronischen Kennungsmitteln zugeordnetes Kommunikationsmodul umfaßt, mit dem beim Auswerten der elektronischen Kennungsmittel die gespeicherten Daten zumindest teilweise aus den elektronischen Kennungsmitteln ausgelesen und wahlweise weitere Daten in den elektronischen Kennungsmitteln gespeichert werden.

19. Verfahren nach Anspruch 17 oder 18, **dadurch gekennzeichnet, daß** beim Auswerten der elektronischen Kennungsmittel Daten aus einem von den elektronischen Kennungsmitteln umfaßten RFID-Chip ausgewertet werden,

20. Verfahren nach mindestens einem der Ansprüche 15 bis 19, **dadurch gekennzeichnet, daß** die Auswertevorrichtung (13) die Kennungsmittel (14) automatisch auswertet, wenn das Nadelmodul (3) an das Antriebsmodul (2) gekoppelt wird.

21. Verfahren nach mindestens einem der Ansprüche 15 bis 20, **dadurch gekennzeichnet, daß** die Kennungsmittel (14) zumindest eine der folgenden, von der Auswertevorrichtung auswertbaren und dem Nadelmodul (3) zuordenbaren Informationen liefern: Seriennummer, für das Nadelmodul (3) nutzbare(s) Antriebsmodul(e), Nutzungsdauer des Nadelmoduls (3), Art der Stechvorrichtung (6) und erlaubte Betriebsparameter wie Stechfrequenz und Betriebsarten.

22. Verfahren nach mindestens einem der Ansprüche 15 bis 21, **dadurch gekennzeichnet, daß** durch die Auswertevorrichtung (23) oder wahlweise die Steuervorrichtung (10) der Betrieb des Handgerätes (1) unterbunden wird, wenn beim Auswerten der Kennungsmittel (14) festgestellt wird, daß das Nadelmodul (3) für eine Nutzung mit dem Antriebsmodul (2) nicht zugelassen ist.

23. Verfahren nach Anspruch 21 oder Anspruch 22, soweit auf Anspruch 21 rückbezogen, **dadurch gekennzeichnet, daß** die Auswertevorrichtung (13) oder wahlweise die Steuervorrichtung (10) den Betrieb des Handgerätes (1) der mindestens einen von der Auswertevorrichtung (13) auswertbaren und dem Nadelmodul (3) zuordenbaren Information entsprechend steuert.
